# EUROPÄISCHE PATENTSCHRIFT

(11) **EP 0 438 703 B1**
(45) Veröffentlichungstag und Bekanntmachung des Hinweises auf die Patenterteilung: **15.06.1994**
(21) Anmeldenummer: 90123993.9
(22) Anmeldetag: 12.12.1990
(51) Int. Cl.: A61M 1/36

(54) **System zur Sammlung und Retransfusion von autologem Blut**
System for the collection and retransfusion of autologous blood
Système pour la récupération et la réinfusion du sang autologue

(30) Priorität: 29.12.1989 DE 3943300
(43) Veröffentlichungstag der Anmeldung: 31.07.1991
(73) Patentinhaber: Werner, Margrit, Dipl.-Ing., D-55129 Mainz (DE); Schütt, Klaus-Hermann, Dr. med., D-55257 Budenheim (DE)
(72) Erfinder: Werner, Margrit, Dipl.-Ing., D-55129 Mainz (DE); Schütt, Klaus-Hermann, Dr. med., D-55257 Budenheim (DE)
(74) Vertreter: Patentanwälte Bartels und Partner

(56) Entgegenhaltungen:
- GB-A- 2 135 890
- GB-A- 2 139 094
- US-A- 3 545 671
- US-A- 4 333 016
- US-A- 4 439 190
- US-A- 4 668 214

## Beschreibung

Die Erfindung betrifft ein System zur Sammlung und Retransfusion von autologem Blut, das die Merkmale des Oberbegriffs des Anspruches 1 aufweist.

Bei einem bekannten Sammel- und Retransfusionssystem, das die Bezeichnung "Solcotrans-System" führt, besteht das Sammelgefäß für das Drainageblut aus einem Beutel, der sich in einer formstabilen Kunststoffflasche befindet, die evakuierbar ist, um in dem Beutel einen Unterdruck erzeugen und für eine gewisse Zeit aufrechterhalten zu können. Vor dem Beginn der Drainage wird in den Beutel eine bestimmte Menge der Antikoagulanzflüssigkeit gegeben. Ist der Beutel im gewünschten Maße gefüllt, wird er von dem zum Drain führenden Schlauch getrennt, damit sein Inhalt dem Patienten über das Mikrofilter retransfundiert werden kann. Ein Nachteil dieses bekannten Systems besteht darin, daß in Abhängigkeit von den Wundverhältnissen eine unterschiedlich stark ausgeprägte Hämolyse entstehen kann, was zu erheblichen Schwankungen in der Qualität der zu retransfundierenden Erythrozyten führt. Ein weiterer Nachteil besteht darin, daß das Blut relativ lange dem Kontakt mit einer Fremdoberfläche ausgesetzt ist, ehe es im Sammelgefäß mit der Antikoagulanzflüssigkeit vermischt werden kann. Dies führt zur Aktivierung von Gerinnungsfaktoren sowie des Kinin- und Komplementsystems. Die Aktivierung der Ca-abhängigen Gerinnungsfaktoren wird erst unterbrochen, wenn das Blut in Kontakt mit dem durch die Antikoagulanzflüssigkeit gebildeten Stabilisator kommt. Eine ausreichende Durchmischung des Blutes mit der Antikoagulanzflüssigkeit ist aber nur dann gewährleistet, wenn Hilfspersonal immer wieder die beiden Flüssigkeiten mechanisch mischt, was ein weiterer Nachteil des bekannten Systems ist. Außer aktivierten Gerinnungsfaktoren kann das im Sammelgefäß enthaltene Blut auch Fett und Fettgewebe aus dem Wundgebiet enthalten. Zwar wird das Blut vor der Transfusion mittels des Mikrofilters mechanisch filtriert. Kleine Gerinnsel unter 40 »l, Fett und aktivierte Gerinnungsfaktoren gelangen aber trotzdem in den Kreislauf des Patienten, so daß das Risiko von thrombembolischen Komplikationen und Fettembolien nicht ausgeschlossen werden kann, was ebenfalls sehr nachteilig ist.

Frei von dem letzgenannten Nachteil ist zwar ein anderes bekanntes System, bei welchem das Blut einem Waschvorgang mit physiologischer Kochsalzlösung unterzogen wird, wodurch Fett und Gerinnungsfaktoren entfernt werden. Dieses System ist aber sehr aufwendig und im Betrieb personalintensiv, weshalb der Einsatz auf große Kliniken beschränkt ist. Hinzu kommt, daß beide bekannte Systeme nicht geschlossen sind, so daß das Risiko einer Kontamination nicht ausgeschlossen ist.

Nicht geschlossen ist auch ein bekanntes Sammel- und Retransfusionssystem der eingangs genannten Art (GB-A-2139094), das zum Zwecke einer interoperativen Absaugung einen Saugstab aufweist, mit dem aus der offenen Wunde nicht nur Blut, sondern zwangsläufig auch Luft angesaugt wird. Außerdem wird dem angesaugten Blut Luft dadurch beigemischt, daß der Saugstab mit einer Öffnung versehen ist, die mehr oder weniger weit mit dem Finger freigegeben wird, um den wirksamen Unterdruck variieren zu können. Antikuagulanzflüssigkeit wird dem Blut bereits im Saugstab beigemischt. Vom Saugstab gelangt das Blut-Luft-Gemisch über eine Leitung in ein Sammelgefäß, in dem ein Unterdruck aufrechterhalten wird, um einerseits das Blut aus der Wunde absaugen zu können und andererseits das Ausscheiden von Luft aus dem Blut zu fördern. Über eine Schlauchleitung gelangt das zu retransfundierende Blut aus dem Sammelgefäß in einen tiefer angeordneten Sammel- und Trennbehälter, der über eine Leitung mit dem Raum über dem Flüssigkeitsspiegel im Sammelgefäß verbunden ist, um weiterhin die Trennung von Luft und Blut zu unterstützen. Über eine in das Innere des als Beutel ausgebildeten Sammel- und Trennbehälters ragende Leitung wird dann das zu retransfundierende Blut einer Kanüle zugeführt.

Der Erfindung liegt die Aufgabe zugrunde, ein System zur Sammlung und Retransfusion von autologem Blut zu schaffen, daß nicht mit den Nachteilen der bekannten Systeme behaftet ist und insbesondere postoperativ sowie auch dort eingesetzt werden kann, wo das bekannte, eine Blutwäsche durchführende System aus Kosten- und Personalgründen nicht einsetzbar ist. Diese Aufgabe löst ein System mit den Merkmalen des Anspruches 1.

Die geschlossene, d.h. das Eindringen von Keimen ausschließende Ausführung des erfindungsgemäßen Systems schließt jegliches Kontaminationsrisiko aus. Dadurch, daß das Blut schon vor dem Eintritt in das Sammelgefäß mit Antikoagulanzflüssigkeit gemischt wird, wird zum einen eine selbsttätige, gute Mischung erreicht und zum anderen die Zeitspanne, in der das Blut dem Kontakt einer Fremdoberfläche ausgesetzt ist, auf ein Minimum reduziert. Vorteilhaft ist ferner, daß mittels der Schlauchpumpe der im Sammelgefäß wirksame Unterdruck gesteuert und dadurch auch auf einem optimalen Wert gehalten werden kann. Ein weiterer, wesentlicher Vorteil wird mittels des Sammel- und Trennbehälters erreicht, in dem, und zwar allein durch Schwerkraft, innerhalb relativ kurzer Zeit eine Trennung des Blutes in Erythrozyten und Plasmabestandteile erfolgt. Es ist deshalb möglich, nur das Erythrozytenkonzentrat über die Retransfusionsleitung dem Patienten zu retransfundieren.

Dieses System ist darüber hinaus geeignet, Plasma- und Erythrozytenaphäresen bei Patienten zum Beispiel mit Hyperviskositätssyndrom, Autoimmunerkrankungen und Hämochromatose bzw. Polycytämie durchzuführen. Bei der Plasmaaphärese erhält der Patient nach Sedimentation des Blutes seine eigene Erythrozyten retransfundiert, das Plasma wird dann über ein konnektiertes Sammelgefäß entsorgt. Bei der Erythrozytenaphärese wird das Erythrozytenkonzentrat verworfen und das Plasma retransfundiert.

Die Trennung der Erythrozyten von den Plasmabestandteilen kann durch Beimischung wenigstens einer Substanz, die eine Senkung der Erythrozyten beschleunigt, z.B. von Hydroxyäthylstärke, zu der Antikoagulanzflüssigkeit beschleunigt werden, da auf diese Weise eine Senkungsbeschleunigung erreicht wird. Hinzu kommt, daß das erfindungsgemäße System es nicht erforderlich macht, zu warten, bis große Mengen an Blut vorhanden sind. Es kann schon nach relativ kurzer Zeit mit der Retransfusion begonnen werden, wodurch vollaktive Erythrozyten retransfundiert werden können. Dabei wird ein Erythrozytenkonzentrat mit einem hohen Hämatokrit transfundiert, was ein Risiko für den Patienten praktisch ausschließt. Da auch ein akzidenteller Keimeintritt ausgeschlossen ist, weil keine Diskonnektierungen innerhalb des Systemes erforderlich sind, sind die Risiken der bekannten Systeme beseitigt. Schließlich bietet das erfindungsgemäße System noch den Vorteil, daß es nicht nur zur postoperativen Absaugung mit Retransfusion, sondern auch zur Wunddrainage und zur interoperativen Absaugung eingesetzt werden kann. Im letztgenannten Falle genügt es, falls eine Absaugung nur an der Endöffnung des Drains erfolgen soll, außen auf den Drain eine Durchführungshülse aufzustecken. Vor dem Wundverschluß wird diese Hülse dann wieder entfernt.

Vorzugsweise ist mit dem Einlaß für die Antikoagulanzflüssigkeit über einen Schlauch ein Tropfkörper verbunden, welcher den Vorrat an Antikoagulanzflüssigkeit, gegebenenfalls gemischt mit wenigstens einer Substanz zur Senkungsbeschleunigung, enthält. Dieser Tropfköper weist vorzugsweise eine Mengendosiereinrichtung auf, die von einem Sensor gesteuert sein kann, der die dem Sammelgefäß zufließende Menge Blut detektiert und dementsprechend volumenabhängig die Beimischung der Antikoagulanzflüssigkeit steuert. Dieser Sensor kann am Eingang des Sammelgefäßes oder davor angeordnet sein. Man kann aber auch das Blut über einen Tropfkörper dem Sammelgefäß zuführen und, abgestimmt auf dessen Mengeneinstellung die Mengendosiereinrichtung für die Antikoagulanzflüssigkeit einstellen.

Das Sammelgefäß ist bei einer bevorzugten Ausführungsform ein formstabiler Behälter, damit in ihm problemlos der gewünschte Unterdruck mit Hilfe der Schlauchpumpe aufrechterhalten werden kann, die an den vom Sammelgefäß zum Sammel- und Trennbehälter führenden Schlauch angesetzt werden kann. Diese Schlauchpumpe kann in Abhängigkeit von dem im Sammelgefäß herrschenden Unterdruck gesteuert werden. Daher weist bei einer bevorzugten Ausführungsform das Sammelgefäß eine Unterdruckanzeigeeinrichtung auf. Diese Unterdruckanzeigeeinrichtung könnte aber auch nur eine optische Anzeige oder ein akustisches Signal erzeugen, damit rechtzeitig die Schlauchpumpe aktiviert wird, falls sie nicht ständig eingeschaltet ist.

Bei einer bevorzugten Ausführungsform weist der Sammel- und Trennbehälter eine Trennzone auf, in welcher der obere Teil, an den die Schlauchleitung angeschlossen ist, dicht gegenüber dem unteren Teil verschließbar ist, an den die Retransfusionsleitung angschlossen ist. Das im unteren Teil angesammelte Erythrozytenkonzentrat kann dann entnommen werden, ohne daß die Gefahr besteht, daß auch das sich im oberen Teil befindende Plasma entnommen wird. Die Trennung in der Trennzone kann beispielsweise mittels einer von außen ansetzbaren Klemmvorrichtung vorgenommen werden. Man kann aber auch eine Verschweißung in der Trennzone vorsehen, sofern für die Retransfusion die nach der erstmaligen Ansammlung an Erythrozytenkonzentrat zur Verfügung stehende Menge ausreichend ist. Das Sammel- und Trennbehälter ist deshalb vorzugsweise ein Beutel, der aus einem weichen, gegebenenfalls verschweißbaren Kunststoff besteht. Ferner kann man statt einer Abtrennung am Auslaß einen Sensor anordnen, der erkennt, ob Erythrozyten oder Plasma abfließt und dann, wenn er keine Erythrozyten mehr detektiert, ein Signal erzeugt oder selbsttätig die Retransfusion beendet.

Für das Sammeln des Erythrozytenkonzentrats ist es vorteilhaft, wenn der untere Teil des Sammel- und Trennbehälters einen sich zum Anschluß der Retransfusionsleitung hin verengenden Trichter bildet.

Die Retransfusionsleitung kann zumindest einen Abschnitt aufweisen, an den eine Schlauchpumpe ansetzbar ist, um das Erythrozytenkonzentrat mit dem notwendigen Druck über das Mikrofilter, das vorzugsweise eine eingebaute Tropfkammer aufweist, dem Patienten zu retransfundieren. Bei dieser Schlauchpumpe kann es sich um diejenige handeln, welche den Inhalt des Sammelgefäßes in den Sammel- und Trennbehälter pumpt. Selbstverständlich kann aber auch, wenn dieser Pumpvorgang ständig erfolgen muß, eine zweite Schlauchpumpe an die Retransfusionsleitung angesetzt werden.

Am Retransfusionsschlauch oder am Auslaß des Sammel- und Trennbehälters kann ein Sensor angeordnet sein, der ein akustisches und/oder optisches Signal erzeugt oder die Schlauchpumpe abschaltet, sobald der Vorrat im Sammel- und Trennbehälter an Erythrozytenkonzentrat aufgebraucht ist. Sofern dieser Sensor Erythrozyten von Plasma unterscheiden kann, kann er, wie oben erwähnt, die Abtrennung des Sammel- und Trennbehälters überflüssig machen.

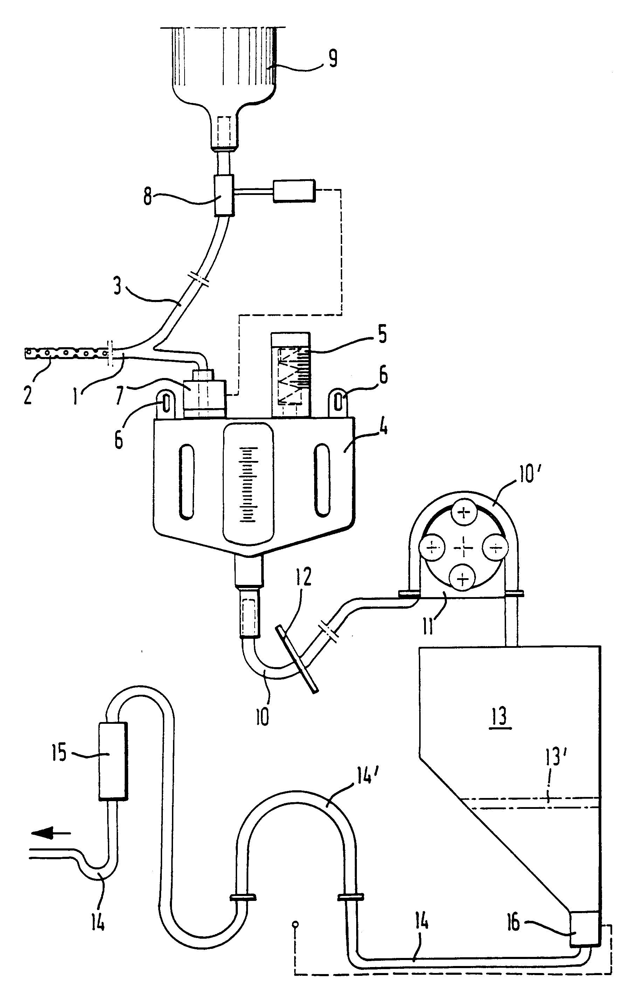

Im folgenden ist die Erfindung anhand eines in der Zeichnung dargestellten Ausführungsbeispiels im einzelnen erläutert. Die einzige Figur zeigt in schematischer Darstellung das Ausführungsbeispiel.

Ein geschlossenes System zur Sammlung und Retransfusion von autologem Blut weist einen aus Kunststoff bestehenden Absaugschlauch 1 auf, dessen einer Endabschnitt als Drain 2 ausgebildet ist. Selbstverständlich wäre es auch möglich, einen separaten Drain vorzusehen, an den der Absaugschlauch 1 angeschlossen ist. In geringem Abstand vom anderen Ende des Absaugschlauches 1 weist dieser einen Abzweig auf, über den ein ebenfalls aus Kunststoff bestehender Tropfschlauch 3 an den Absaugschlauch 1 angeschlossen ist. Mit letzterem kann der Tropfschlauch 3 einstückig ausgebildet sein oder verschweißt sein. Die Verbindung im Bereich des Abzweiges kann aber auch mittels eines Verbindungsstückes hergestellt sein.

Das dem Abzweig benachbarte Ende des Absaugschlauches 1 ist an ein formstabiles Sammelgefäß 4 aus Kunststoff angeschlossen, und zwar im Bereich von dessen Oberseite. An dieser Oberseite weist das Sammelgefäß 4 ferner ein Unterdruckanzeigegerät 5 auf, welches in Prozenten des Vakuums den im Sammelgefäß 4 herrschenden Unterdruck anzeigt. Zwei Ösen 6 an der Oberseite des Sammelgefäßes 4 ermöglichen es, das Sammelgefäß 4 an eine Haltevorrichtung anzuhängen.

Dort, wo der Absaugschlauch 1 und das Sammelgefäß 4 angeschlossen ist, ist ein Sensor 7 angeordnet, welcher die Flüssigkeitsmenge, evtl. korrigiert mit dem Anteil der dem Blut zudosierten Substanz, detektiert, welche durch den Absaugschlauch 1 in das Sammelgefäß 4 fließt. Dieser Sensor 7 steuert in Abhängigkeit von der detektierten Flüssigkeitsmenge eine Mengendosiereinrichtung 8, die am Ausgang eines Tropfkörpers 9 angeordnet ist und die Flüssigkeitsmenge steuert, die der in bekannter Weise ausgebildete Tropfkörper 9 abgibt, an den der Tropfschlauch 3 angeschlossen ist.

Mit einem Auslaß am unteren Ende des Sammelgefäßes 4 ist das eine Ende einer Schlauchleitung 10 verbunden, welche mit Ausnahme eines Abschnittes 10', der aus einem Silikongummischlauch besteht, aus Kunststoff besteht. An den Abschnitt 10' ist eine auch als Rollenpumpe bezeichnete Schlauchpumpe 11 ansetzbar. Zwischen dem Abschnitt 10' und dem Sammelgefäß 4 ist die Schlauchleitung 10 mittels einer Schlauchklemme 12 abklemmbar.

Das andere Ende der Schlauchleitung 10 ist oben an einen aus einem weichen Kunststoff bestehenden Sammel- und Trennbeutel 13 angeschlossen, der sich in seiner unteren Hälfte zu dem an seinem unteren Ende vorgesehenen Auslaß hin trichterförmig verjüngt. An diesen Auslaß ist eine Retransfusionsleitung 14 angeschlossen, die wie die Schlauchleitung 10 einen Abschnitt 14' aus Silikongummi aufweist, an den die Schlauchpumpe 11 oder eine andere Schlauchpumpe ansetzbar ist. Im übrigen besteht die Retransfusionsleitung 14 ebenfalls aus Kunststoff.

Zwischen dem Abschnitt 14' und dem mit einer Transfusionskanüle zu verbindenden Ende liegt im Zuge der Retransfusionsleitung 14 ein bekanntes Mikrofilter 15 mit Tropfkammer.

Für eine Retransfusion enthält der Tropfkörper 9 eine Antikoagulanzflüssigkeit, der vorzugsweise ein Senkungsbeschleunigungsmittel, z.B. ein Hydroxyäthylstärke-Citrat, beigemischt ist. Die Blutabsaugung über den Absaugschlauch 1 in das Sammelgefäß 4 erfolgt aufgrund des hier mit Hilfe der Schlauchpumpe 11 erzeugten Unterdruckes. Der Unterdruck kann durch Ein- und Ausschalten der Schlauchpumpe 11 und/oder durch eine Steuerung von deren Drehzahl stets auf einem optimalen Wert gehalten werden. Eine wundgesteuerte Vakuumeinstellung setzt die Erythrozyten weniger großen Scherkräften bei der Absaugung aus, was das Risiko einer Qualitätminderung der Erythrozyten durch Hämolyse ausschließt. Entsprechend der vom Sensor 7 detektierten Menge an Blut wird das Antikoagulanz-Senkungsbeschleunigungsmittelgemisch so zudosiert, daß beispielsweise zehn Teile Blut mit drei Teilen eines Hydroxyäthylstärke-Citratgemisches gemischt werden. Da die Zudosierung bereits im Absaugschlauch 1 erfolgt, ist eine gute Durchmischung gewährleistet. Der Inhalt des Sammelgefäßes 4 braucht also nicht mehr nachträglich durchmischt zu werden. Ein weiterer Vorteil der Zufügung des Senkungsbeschleunigungsmittels zu dem Blut bereits im Absaugschlauch 1 besteht darin, daß die Zeit, während deren das Blut in Kontakt mit Fremdoberflächen ist, sehr gering ist. Dadurch wird eine Aktivierung von Gerinnungsfaktoren sowie eine Aktivierung des Kinin- und Komplementsystems zumindest weitgehend vermieden. In der Regel wird es vorteilhaft sein, bei intraoperativer Absaugung das Blut nur über die am Ende des Absaugschlauches 1 vorhandene Öffnung abzusaugen. In diesem Falle wird über den Drain 2 eine Hülse geschoben, welche die in der Wandung vorhandenen Öffnungen abdeckt. Diese Hülse wird vor dem Wundverschluß entfernt.

Auch bei postoperativer Absaugung arbeitet das erfindungsgemäße System in der zuvor beschriebenen Weise, wenn dabei eine Retransfusion durchgeführt werden soll. Aber auch dann, wenn anschließend nur noch eine Wunddrainage erforderlich ist, kann das erfindungsgemäße System benutzt werden. Es brauchen dann nur der Tropfschlauch 3 sowie die Retransfusionsleitung 14, letztere zweckmäßigerweise möglichst nahe am Auslaß des Sammel- und Trennbeutels 13, abgeklemmt oder zugeschweißt, vorzugsweise abgeschweißt zu werden. Der Sammel- und Trennbeutel 13 dient dann als Sammelbeutel für das abgesaugte Wundsekret. Dabei ist von besonderem Vorteil, daß das Drainagesystem stets geschlossen ist. Ein Austausch des Sammelgefäßes 4 ist nicht erforderlich, weil dessen Unterdruck mittels der Schlauchpumpe 11 immer wieder regeneriert werden kann.

## Patentansprüche

1. System zur Sammlung von autologem Blut für eine Retransfusion mit
a) einem Sammelgefäß (4) zur Aufnahme von Blut und einer Antikoagulanzflüssigkeit, das an einen zu einem rohrförmigen Ansaugteil (2) führenden Schlauch (1) angeschlossen ist und in dem Unterdruck aufrechterhalten werden kann,
b) einem Einlaß für die Koagulanzflüssigkeit zwischen dem Sammelgefäß (4) und dem offenen Ende des Ansaugteils (2),
c) einem Sammel- und Trennbehälter (13), der über eine Schlauchleitung (10) mit einem Auslaß des Sammelgefäßes (4) verbunden ist und einen Auslaß für die zu retransfundierende Flüssigkeit hat,
dadurch gekennzeichnet, daß
d) für eine postoperative Absaugung das System geschlossen und der Ansaugteil als Drain (2) ausgebildet ist,
e) der Einlaß für die Antikoagulanzflüssigkeit zwischen dem Drain (2) und dem Sammelgefäß (4) vorgesehen ist,
f) am unteren Ende des Sammel- und Trennbehälters (13) dessen Auslaß (16) für eine Entnahme des sich absetzenden Erythrozytenkonzentrates vorgesehen ist, und
g) zur Aufrechterhaltung des Unterdrucks im Sammelgefäß (4) eine an die vom Sammelgefäß (4) zum Sammel- und Trennbehälter (13) führende Schlauchleitung (10) ansetzbare Schlauchpumpe (11) vorgesehen ist.

2. System nach Anspruch 1, dadurch gekennzeichnet, daß an den Auslaß (16) des Sammel- und Trennbehälter (13) eine ein Microfilter (15) enthaltende Retransfusionsleitung (14, 14') angeschlossen ist.

3. System nach Anspruch 1 oder 2, dadurch gekennzeichnet, daß mit dem Einlaß für die Antikoaguzlanzflüssigkeit über einen Schlauch (3) ein den Vorrat an Antikoagulanzflüssigkeit enthaltender Tropfkörper (9) verbunden ist.

4. System nach Anspruch 3, dadurch gekennzeichnet, daß der Tropfkörper (9) eine Mengendosiereinrichtung (8) aufweist, die von einem Sensor (7) gesteuert ist, der ein die zufließende Blut-Menge repräsentierendes Steuersignal erzeugt.

5. System nach Anspruch 4, dadurch gekennzeichnet, daß der Sensor (7) am Eingang des Sammelgefäßes (4) oder davor angeordnet ist.

6. System nach einem der Ansprüche 1 bis 5, dadurch gekennzeichnet, daß das Sammelgefäß (4) ein formstabiler Behälter ist.

7. System nach Anspruch 6, dadurch gekennzeichnet, daß das Sammelgefäß (4) eine Unterdruckanzeigeeinrichtung (5) aufweist.

8. System nach einem der Ansprüche 1 bis 7, dadurch gekennzeichnet, daß der Sammel- und Trennbehälter (13, 13') eine Trennzone (13') aufweist, in welcher der obere Teil, an den die Schlauchleitung (10, 10') angeschlossen ist, dicht gegenüber dem unteren Teil verschließbar ist, der an seinem unteren Ende den Auslaß (16) für das Erythrozytenkonzentrat aufweist.

9. System nach Anspruch 8, dadurch gekennzeichnet, daß der Sammel- und Trennbehälter (13, 13') ein Beutel ist.

10. System nach Anspruch 9, dadurch gekennzeichnet, daß der Beutel aus einem verschweißbaren Kunststoff besteht.

11. System nach einem der Ansprüche 8 bis 10, dadurch gekennzeichnet, daß der untere Teil des Sammel- und Trennbehälters (13, 13') einen sich zum Auslaß (16) hin verengenden Trichter bildet.

12. System nach einem der Ansprüche 2 bis 11, dadurch gekennzeichnet, daß die Retransfusionsleitung (14, 14') zumindest einen Abschnitt (14') aufweist, an den eine Schlauchpumpe (11) ansetzbar ist.

13. System nach Anspruch 12, dadurch gekennzeichnet, daß an der Retransfusionsleitung (14, 14') oder dem Auslaß des Sammel- und Trennbehälters ein Sensor (16) angeordnet ist, der die an die Retransfusionsleitung (14, 14') angesetzte Schlauchpumpe abschaltat, wenn der Vorrat im Sammel- und Trennbehälter (13, 13') an Erythrozytenkonzentrat aufgebraucht ist, und/oder ein optisches und/oder akustisches Signal erzeugt.

## Claims

1. System for collecting autologous blood for retransfusion, having:
a) a collecting vessel (4) for receiving blood and an anti-coagulant fluid which is connected to a hose (1) leading to a tubular intake part (2) and in which negative pressure can be maintained;
b) an inlet for the coagulant fluid between the collecting vessel (4) and the open end of the intake part (2);
c) a collecting and separating container (13) which is connected by means of a hose-line (10) to an outlet of the collecting vessel (4) and has an outlet for the fluid to be retransfused;
characterised in that:
d) the system is closed for post-operative drawing-off and the intake part is in the form of a drain (2);
e) the inlet for the anti-coagulant fluid (4) is provided between the drain (2) and the collecting vessel (4);
f) the outlet (16) of the collecting and separating container (13) for tapping the settling erythrocyte concentrate is provided at the lower end of the collecting and separating container (13); and
g) a hose pump (11) which can be connected to the hose-line (10) leading from the collecting vessel (4) to the collecting and separating container (13) is provided for maintaining the negative pressure in the collecting vessel (4).

2. System according to Claim 1, characterised in that a retransfusion line (14, 14') containing a microfilter (15) is connected to the outlet (16) of the collecting and separating container (13).

3. System according to Claim 1 or Claim 2, characterised in that a drip supply (9) containing the supply of anti-coagulant fluid is connected by means of a hose (3) to the inlet for the anti-coagulant fluid.

4. System according to Claim 3, characterised in that the drip supply (9) comprises a metering device (8) which is controlled by a sensor (7) which generates a control signal representing the inflowing amount of blood.

5. System according to Claim 4, characterised in that the sensor (7) is disposed at the inlet of the collecting vessel (4) or in front thereof.

6. System according to any one of Claims 1 to 5, characterised in that the collecting vessel (4) is a dimensionally stable container.

7. System according to Claim 6, characterized in that the collecting vessel (4) comprises a negative pressure display device (5).

8. System according to any one of Claims 1 to 7, characterised in that the collecting and separating container (13, 13') has a separation zone (13') in which the upper part, to which the hose-line (10, 10') is connected, can be tightly sealed with respect to the lower part which comprises the outlet (16) for the erythrocyte concentrate at its lower end.

9. System according to Claim 8, characterised in that the collecting and separating container (13, 13') is a bag.

10. System according to Claim 9, characterised in that the bag is made of a plastics material which can be heat-sealed.

11. System according to any one of Claims 8 to 10, characterised in that the lower part of the collecting and separating container (13, 13') forms a funnel which tapers towards the outlet (16).

12. System according to any one of Claims 2 to 11, characterised in that the retransfusion line (14, 14') comprises at least one portion (14') to which a hose pump (11) can be connected.

13. System according to Claim 12, characterised in that there is disposed on the retransfusion line (14, 14') or at the outlet of the collecting and separating container a sensor (16) which switches off the hose pump connected to the retransfusion line (14, 14') when the supply of erythrocyte concentrate in the collecting and separating container (13, 13') is used up and/or generates an optical and/or acoustic signal.

## Revendications

1. Système de récupération de sang autologue pour une retransfusion, comportant :
a) une cuvette collectrice (4) pour recevoir le sang et un liquide anticoagulant, qui est reliée à un tuyau souple (1) aboutissant à une partie d'aspiration (2) de forme tubulaire et qui peut être maintenue en dépression,
b) une entrée pour le liquide anticoagulant,qui est située entre la cuvette collectrice (4) et l'extrémité ouverte de la partie d'aspiration (2),
c) un récipient collecteur et séparateur (13), qui est relié par l'intermédiaire d'un tuyau souple (10) à une sortie de la cuvette collectrice (4) et qui comporte une sortie pour le liquide à retransfuser,
- caractérisé en ce que :
d) pour une aspiration post-opératoire, le système est fermé et la partie d'aspiration est agencée comme un drain (2),
e) l'entrée pour le liquide anticoagulant est située entre le drain (2) et la cuvette collectrice (4),
f) à l'extrémité inférieure du récipient collecteur et séparateur (13), sa sortie (16) est prévue pour une décharge du concentrat d'érythrocytes qui se dépose, et
g) pour un maintien de la dépression dans la cuvette collectrice (4), il est prévu une pompe à tuyau souple (11), pouvant être mise en place sur le tuyau souple (10) s'étendant de la cuvette collectrice (4) au récipient collecteur et séparateur (13).

2. Système selon la revendication 1, caractérisé en ce qu'à la sortie (16) du récipient collecteur et séparateur (13) est raccordé un conduit de retransfusion (14, 14') contenant un microfiltre (15).

3. Système selon la revendication 1 ou 2, caractérisé en ce qu'à l'entrée pour le liquide anticoagulant est raccordé, par l'intermédiaire d'un tuyau souple (3), un distributeur goutte à goutte (9) contenant la réserve de liquide anticoagulant.

4. Système selon la revendication 3, caractérisé en ce que le distributeur goutte à goutte (9) comporte un dispositif de dosage quantitatif (8), qui est commandé par un capteur (7), qui produit un signal de commande représentant la quantité de sang distribuée.

5. Système selon la revendication 4, caractérisé en ce que le capteur (7) est disposé à l'entrée de la cuvette collectrice (4) ou avant celle-ci.

6. Système selon une des revendications 1 à 5, caractérisé en ce que la cuvette collectrice (4) est un récipient de forme stable.

7. Système selon la revendication 6, caractérisé en ce que la cuvette collectrice (4) comporte un dispositif d'indication de dépression.

8. Système selon une des revendications 1 à 7, caractérisé en ce que le récipient collecteur et séparateur (13, 13') comporte une zone de séparation (13') dans laquelle la partie supérieure, à laquelle est raccordé le tuyau souple (10, 10'), peut être obturée de façon étanche par rapport à la partie inférieure, qui comporte à son extrémité inférieure la sortie (16) pour le concentrat d'érythrocytes.

9. Système selon la revendication 8, caractérisé en ce que le récipient collecteur et séparateur (13, 13') est une poche.

10. Système selon la revendication 9, caractérisé en ce que la poche se compose d'une matière plastique soudable.

11. Système selon une des revendications 8 à 10, caractérisé en ce que la partie inférieure du récipient collecteur et séparateur (13, 13') constitue un entonnoir se rétrécissant vers la sortie (16).

12. Système selon une des revendications 2 à 11, caractérisé en ce que le conduit de retransfusion (14, 14') comporte au moins une partie (14') contre laquelle peut être mise en place une pompe à tuyau souple (11).

13. Système selon la revendication 12, caractérisé en ce que sur le conduit de retransfusion (14, 14' ) ou à la sortie du récipient collecteur et séparateur est disposé un capteur (16), qui arrête la pompe à tuyau souple mise en place sur le conduit de retransfusion (14, 14') quand la réserve de concentrat d'érythrocytes dans le récipient collecteur et séparateur (13, 13') est épuisée et/ou qui produit un signal optique et/ou acoustique.
